# EUROPEAN PATENT SPECIFICATION

(11) **EP 4 188 273 B1**
(45) Date of publication and mention of the grant of the patent: **19.11.2025**
(21) Application number: 20949648.8
(22) Date of filing: 31.12.2020
(51) Int. Cl.: A61C 7/10, A61C 8/00

(54) **A DEVICE FOR UPPER JAW EXPANSION**
VORRICHTUNG ZUR OBERKIEFEREXPANSION
DISPOSITIF D'EXPANSION DE MÂCHOIRE SUPÉRIEURE

(30) Priority: 13.08.2020 TR 202012739
(43) Date of publication of application: 07.06.2023
(73) Proprietor: Alev, Yüksel, Konak/Izmir (TR)
(72) Inventor: Alev, Yüksel, Konak/Izmir (TR)
(74) Representative: Yamankaradeniz, Kemal
(86) International application number: PCT/TR2020/051479
(87) International publication number: WO 2022/035392

(56) References cited:
- CN-A- 102 462 551
- CN-A- 105 832 433
- CN-A- 107 520 646
- CN-A- 111 437 052
- CN-U- 208 942 436
- DE-A1- 102018 114 830
- KR-A- 20160 133 921
- KR-B1- 102 088 353
- US-A- 5 575 643
- US-A1- 2009 130 620

## Description

### Technical Field

The invention relates to a device used in orthodontic treatments in the field of orthodontics which is a branch of dentistry.

The invention specifically relates to a device which provides ease and comfort in application by being used in the maxillary expansion procedures in cases of narrow maxilla in addition to various cases where it is required to use jaw bones as a support for anchorage.

### State of Art

Devices used in maxillary expansion procedures in orthodontic treatments are generally divided into three main groups. These devices are as follows: tooth borne (Tooth Borne Rapid Palatal Expansion (RPE); tooth and bone (hybrid) borne (Tooth - Bone Borne (Hybrid) Rapid Palatal Expansion (RPE); and only bone borne (Bone Borne Rapid Palatal Expansion (RPE). Mini-screws are used in implementations of hybrid borne and only bone borne devices.

The procedures performed in order to use the bone borne expansion devices in current implementations are as follows; in the beginning of the orthodontic treatment, at the first appointment initially diagnostic impressions and x-ray films are obtained from the patient as it is done for every orthodontic patient. In some cases, CB-CT images might be required. Afterwards, the method and materials to be used are selected. Diameter and length values for the mini-screws for a proper anchorage into the bone are determined. Expansion device (Expander) is used as force resource. The said expansion devices are manufactured in different sizes depending on their capacity of expansion. After the necessary evaluation and analysis are performed, one of these expanders is chosen according to the upper jaw narrowness and the needed rate of expansion, and the mini-screws to be used are determined.

The patient is called to the clinic for a second appointment to place the mini-screws to the bone of the maxilla. The mini-screws are placed according to the well-known general framework of information and rules and are inserted into the bone by straight eye. Sometimes, in order to provide more precise placement, additional guide plates are prepared in laboratories. In the insertion procedure, attention must be paid to the anatomical structures such as sinus, dental roots, nasal cavity, and neurovascular bundle in the area. Therefore, the placement of mini-screws is a procedure which requires an experienced practitioner. There is a need for abutments to enable the connection of the mini-screws to the expander. These abutments are connected to the micro screws via a fixation screw while they are attached to the expander by soldering.

Connecting pieces in the form of cap are used in order to transfer the position and direction of the inserted mini-screws to the laboratory environment. The said transfer caps are placed in a semi-fixed manner to the head part of the mini-screw and additional measurements should be taken in order to prevent them to fall and be swallowed. Detailed impression is taken from the maxilla by precision impression material in a manner that the caps are positioned inside.

In laboratory, parts which look like the head design of the mini-screws (analogs) are placed over the transfer caps which are involved into the obtained impression. Casted plaster model is acquired in a manner that the laboratory analogs stay inside the cast. The abutment is placed to the head part of the analog and it is tightly fixed via a fixation screw. A screwdriver which is appropriate for this fixation screw is used for this procedure. By this procedure, since the abutments in the plaster cast are located as they should be on the maxilla in the palate area in the expected manner, the arms of the expander are being adapted to their position and soldering is performed. The soldering must be durable because the force applied by the expander needs to be transferred as it is to the abutments, to the mini-screws and ultimately to the bone.

In order to apply the prepared expansion device, the patient is called to the clinic once more. The device is placed onto the mini-screws and is tightly affixed via the fixation screws. This procedure is performed by small screwdriver with appropriate screwdriver blade or with a screwdriver blade coupled with the Contra-angle Handpiece. The angles of placement between the mini-screws which are placed in the prior session must be close to parallel. In case that there is a great deviation, difficulties can be experienced when placing the low flexible abutments and the expansion device soldered onto them. Depending on the need, the number of mini-screws can be two, four or six. The need for a precise performance increases as their number increases, in terms of obtaining a coherence of angles. Therefore, this point must be considered prior by the practitioner. The next step is activation of the expander within the mouth. By this activation, it is enabled that the created force is transferred onto bone via the abutments, the fixation screws and the mini-screws. As a result of this, the upper jaw starts to expand, and the maxilla expands more and more periodically with each new activation.

Each step and each additional part in the current application which is described above cause waste of time and material consumption; while it also creates an error risk since it requires an application depended on the experience of the practitioner. In the said application, precision impression materials with high costs are used and there is a risk of for some small parts to be swallowed. Since each device must be produced in a manner to be tailored for each patient, a laboratory work is required, which increases both the cost and time needed. For the said application, which requires the experience of the practitioner, it is a must for her/his to receive some additional trainings. In addition, there is a risk of trauma regarding to the anatomical structures which are around the area of the application.

As an example of the current state of art, the document with number US2009081614 A1, which is found in the conducted literature search, can be given. The said document relates to an orthodontic implant system. In the invention, an orthodontic implant system with a screw implant of elongated body comprising a thread first section for anchoring in a jaw bone, a second section with screw implant for protruding from the jaw bone and consisting of a detachable head to be joined to the screw implant is mentioned. Each step and each additional part in the application of the said orthodontic implant system cause time and material consumption; while it also creates a margin of error risk due to the fact that it requires an application depended on the experience of the practitioner.

KR 102 088 353 B1 relates to a maxillary bone expansion device, and more particularly, to a maxillary bone expansion device that can accurately perform the placement and position of a mini screw.

KR 2016 0133921 A relates to a device for expanding a palatal bone to the left or right for orthodontic treatment, snoring treatment or sleep apnea treatment.

US 2009/130620 A1 related to bone supported palatal expansion appliance and utilizes a modified expansion screw device with two closely approximated parallel activation rods that are both textured along their length and nonround in cross section

DE 10 2018 114830 A1 relates to a palate extension device for orthodontic treatment.

As a consequence, a development in the respective technical field is required due to the problems described above and the insufficiency of the current solutions.

### Aim of the Invention

The present invention relates to a device for upper jaw expansion which removes the disadvantages mentioned above and which provides new advantages to the respective field.

The main aim of the invention is to provide a device which ensures ease and comfort in application by being used in the upper jaw expansion procedures in cases of maxilla narrowness in addition to various cases where it is required to use jaw bones for support.

The aim of the invention is to provide a device which does not cause a risk of trauma for the anatomical structures which are located around the area of application.

Another aim of the invention is to provide a device which does not require the experience of the practitioner for the proper placement during the application.

Another aim of the invention is to provide a device which eliminates the adjustment steps, the any needs of preparation steps, and the any need of laboratory procedure phase for the application.

Another aim of the invention is to provide a device which reduce the required time by half allowing implementation of mini-screws and the device together in one session, instead of two sessions.

Another aim of the invention is to provide a device which eliminates the stress of the patient and difficulties of application.

In order to fulfill all the aims which are described above and can be inferred from the detailed description, the present invention is as defined in claim 1.

The structural and characteristic features of the invention and all its advantages will be understood more clearly through the figures provided below and detailed descriptions with reference to these figures. Therefore, the evaluation should be made based on these figures and detailed descriptions.

### Brief Description of Drawings

- Figure 1: is the perspective view of the device of the invention.

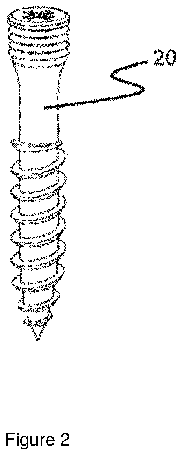

- Figure 2: illustrates the mini-screw of the device of the invention.
- Figure 3: illustrates the guide tube of the device of the invention.
- Figure 4: is a sectional view of the mini-screw of the device of the invention where it is fixed into the bone.

### Description of References

- 10a.: Expander's flat body
- 101.: Body locking hole
- 11.: Extending Screw
- 12.: Adjustment member (Keyhole)
- 13.: Pin
- 20.: Mini-screw
- 30.: Guide tube
- 31.: Head of guide tube

### Detailed Description of the Invention

In this detailed description, the alternatives of the device of the invention are described only for a better understanding of the subject matter without imposing any limitations.

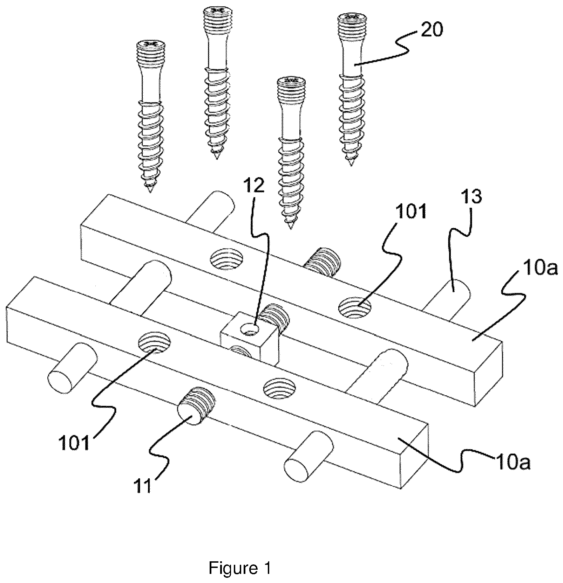

- In Figure 1, the general view of the device of the invention is given. According to this, the device fundamentally comprises two expander's flat bodies (10a) (Figure 1) which are positioned next to each other and have body locking holes (101) with inner threads passing from its upper surface to its lower surface, an extending screw (11) that lies between the expander's flat bodies (10a) and enables the adjustment of the distance of the expander bodies (10) via the integrally formed keyhole adjustment member (12), a pin (13) which is located on both sides of the expander's flat bodies (10a) and which guides movements of the expander's flat bodies (10a), a mini-screw (20) which enables the expander's flat bodies (10a) to be fixed into the upper jaw palate by being fastened to the locking holes (101).

Expander's flat body (10a), which constitutes the main structure of the device of the invention, generally has a rectangular form and there are body locking holes (101) with inner threads located with a certain distance on its upper surface.

An extending screw (11) is inserted in between two expander's flat bodies (10a) (Figure 1) which are located next to each other and the distance between the expander's flat bodies (10a) regarding each other is adjusted by a keyhole adjustment member (12) which is integrally located on the extending screw (11). By this way, by adjusting the distance between the expander's flat bodies (10a) on the upper jaw palate area, the force which is required for the maxillary expansion procedure is obtained.

On both sides of the expander's flat bodies (10a), pins (13) which guide movements of the expander's flat bodies (10a) are inserted.

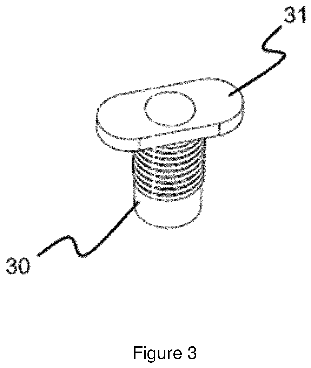

The head part of the mini-screw (20), which enables the expander's flat bodies (10a) to be fixed onto the upper jaw palate has outer threads as shown in Figure 3, and it has a structure suitable to the locking hole (101) on the expander's flat bodies (10a).

As seen in Figure 3, the guide tube (30) is fundamentally a pipe with threads on its outer surface and it has a head (31) part of which the upper surface expands flatly towards the sides. The guide tube (30) is used for creating a guiding path on palate by using of a guiding bone drill before the application, in order to provide that the mini-screws (20) enter into the bone with a correct angle. The guide tubes (30) are screwed into the body locking holes (101) located on the expander's flat bodies (10a) via the threads on its outer surface and after creating the guiding path on the palate by using the appropriate bone drill through the chamber of the guide tube (30) they are unscrewed. By this way, it is provided that the mini-screws (20) are placed into the bone with a proper angle due to the preliminary preparation performed on the bone by the help of the guide tubes (30).

*The application of the device of the invention is as follows;*

In order to be able to apply the device of the invention, firstly, the upper jawbone is evaluated with different angles and depth values by the help of the CB-CT films and the most efficient location of usage on the upper jawbone is determined according to these information. Choosing of the device and its positioning is performed by using the casted plaster model in case of conventional taken impression, or by using the 3D printed model in case the impression is taken by three-dimensional scanning. This measurement is integrated to the CBCT image by the help of certain landmarks. If a digital model is created by intraoral scanning, this procedure is directly performed in a similar manner. The directions, depths, and diameters of the mini-screws (20) are determined and planned via the software.

The position of the body locking holes (101) on the expander's flat bodies (10a) of the device determine the angles and depths of the mini-screws (20) by guiding those mini-screws (20).

The bone which is located nearly to the 2 mm right and to the 2 mm left of the middle line of the palate is preferred. In this preference, body locking holes (101) on the expander's flat bodies (10a) are used.

The correct positioning of the device on the upper jaw palate of the patient is determined by the basic pink wax or thermoforming sheet which serves the role of positioning. This sheet of wax also is helpful in terms of adjusting the distance between the gingiva in the palate and the device. To obtain the correct angle, the guide tubes (30) are screwed into the body locking holes (101) on the expander's flat bodies (10a) and after creating a guiding path on the palate through the chamber of the guide tube (30) by using the appropriate guide bone drill, the preliminary preparation on the bone is completed by unscrewing the guide tubes (30).

Due to the preliminary preparation on the bone, the mini-screws (20) are inserted into the bone with a correct angle by being fastened to the body locking holes (101) on the expander's flat bodies (10a) and they are ultimately screwed to the body locking holes (101) in the final rotation cycle. Thanks to compatible structural connection of the outer threads on the head part of the mini-screw (20) to the inner threads of the body locking holes (101) of the expander's flat bodies (10a), power resource from the expander is directly transferred to the bone continuously and without any deviation in the direction of the force. In addition to this, due to the fact that the outer threads on the head part of the mini-screw (20) are structurally compatible to the inner threads of the body locking holes (101), the device is fixed within a certain distance from the soft tissue in the palate area by the mini-screws (20), and negative effects such as compression, crushing, cutting or pressing during the activation (distance adjustment) with the extending screw (11) are prevented.

After a temporary adhesion is performed between the device and the positioning wax, the correct position in the mouth is determined by the help of the surrounding teeth (1. and 2. premolars) onto which the sheet extends and is supported by. If a digital planning is performed by CB-CT, a special custom made plastic based guide plate (Surgical Guide) can be prepared and positioning wax is not needed in this case.

The established correct position of the device on the palate both determines the insertion direction of the mini-screws (20) to the bone and it also enables the mini-screws (20) to hold tightly onto the inner threads of the body locking holes (101) by the virtue of the outer threads on their head part, after reaching a certain depth.

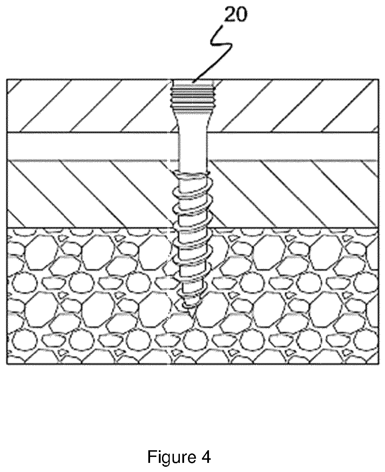

As seen in Figure 4, the final position of the device on palate is determined by placing two mini-screws (20) into the bone. Since there is no need for a positioning wax or guide plate for positioning the other two micro screws (20), the said wax or the plate is easily loosened by a tool and is displaced from its current location. By positioning and fastening the other two mini-screws (20) in the correct position, all of the mini-screws (20) are being fixed.

The expander's flat bodies (10a) are completely fixed onto the palate by the mini-screws (20) being screwed to the body locking holes (101) and they become ready for the expansion procedure.

By periodically activating of the extending screw (11), via the keyhole adjustment member (12), which lies between the expander's flat bodies (10a) the force which is created due to the increasing distance between the expander's flat bodies (10a) or with regard to each other, is transferred to the bone and the maxilla expands.

The device of the invention can be used in various procedures, where it is required to use the palate bone as support, such as Hybrid RME, Distalization, Mesialization, Intrusion, Extrusion, Protrusion, Retraction, Retention etc., with certain modifications in order to provide an advantage and ease in the procedure.

## Claims

1. An upper jaw expander for use in orthodontic treatments, **comprising:**
- a pair of expander's flat bodies (10a) which are configured to be placed next to each other, wherein each flat body (10a) has two locking holes (101) with inner threads passing from its upper surface to its lower surface,
- an extending screw (11) which goes through the expander's flat bodies (10a) and which enables the arrangement of the distance between the expander's flat bodies (10a) via an integrally formed keyhole adjustment member (12),
- a pair of pins (13) inserted in the expander's flat bodies (10a) in a way that the extending screw (11) is positioned between the pins (13) and configured to guide the movements of the expander's flat bodies (10a),
- four mini-screws (20), each mini screw (20) with a head part and outer threads on said head part which enables the expander's flat bodies (10a) to be fixed onto the upper jaw palate by being fastened to the inner threads of the locking holes (101), wherein each body locking hole (101) is located between a pin (13) and the extending screw (11);
- a guide tube (30) which has outer threads on its outer surface in order to be screwed into the body locking holes (101) for creating a guiding path on the palate by using an appropriate bone drill and then unscrewed, and which has a head (31) part of which the upper surface flatly expands to the sides.

2. The device according to claim 1, **characterized by** the pair of expander's flat bodies (10a) being configured to be connected to the bone located on the right and left with a determined distance from the middle line of the palate via the body locking holes (101) and the mini-screws (20).

## Patentansprüche

1. Oberkieferexpander zur Verwendung bei kieferorthopädischen Behandlungen, **umfassend:**
- ein Paar flacher Körper (10a) des Expanders, die dazu konfiguriert sind, nebeneinander platziert zu werden, wobei jeder flache Körper (10a) zwei Verriegelungslöcher (101) mit Innengewinden aufweist, die von seiner oberen Oberfläche zu seiner unteren Oberfläche verlaufen,
- eine Verlängerungsschraube (11), die durch die flachen Körper (10a) des Expanders geht und welche die Anordnung des Abstands zwischen den flachen Körpern (10a) des Expanders über ein einstückig gebildetes Schlüssellocheinstellelement (12) ermöglicht,
- ein Paar Stifte (13), die in die flachen Körper (10a) des Expanders in einer Weise eingesetzt sind, dass die Verlängerungsschraube (11) zwischen den Stiften (13) positioniert ist, und dazu konfiguriert, die Bewegungen der flachen Körper (10a) des Expanders zu führen,
- vier Minischrauben (20), jede Minischraube (20) mit einem Kopfteil und Außengewinden an dem Kopfteil, das ermöglicht, die flachen Körper (10a) des Expanders an dem Oberkiefergaumen zu fixieren, indem sie an den Innengewinden der Verriegelungslöcher (101) befestigt werden, wobei sich jedes Körperverriegelungsloch (101) zwischen einem Stift (13) und der Verlängerungsschraube (11) befindet;
- ein Führungsrohr (30), das Außengewinde an seiner Außenoberfläche aufweist, um durch Verwenden eines angemessenen Knochenbohrers in die Körperverriegelungslöcher (101) zum Schaffen eines Führungsweges an dem Gaumen eingeschraubt und dann abgeschraubt zu werden, und das einen Kopf (31) aufweist, von dem ein Teil die obere Oberfläche flach zu den Seiten expandiert.

2. Vorrichtung nach Anspruch 1, **dadurch gekennzeichnet, dass** das Paar flacher Körper (10a) des Expanders dazu konfiguriert ist, mit dem Knochen, der sich rechts und links befindet, mit einem bestimmten Abstand von der Mittellinie des Gaumens über die Körperverriegelungslöcher (101) und die Minischrauben (20) verbunden zu sein.

## Revendications

1. Dispositif d'expansion de mâchoire supérieure destiné à être utilisé dans des traitements orthodontiques, **comprenant** :
- une paire de corps plats (10a) de dispositif d'expansion qui sont conçus pour être placés l'un à côté de l'autre, chaque corps plat (10a) possédant deux trous de verrouillage (101) avec des filets internes passant de sa surface supérieure à sa surface inférieure,
- une vis d'extension (11) qui traverse les corps plats (10a) du dispositif d'expansion et qui permet l'agencement de la distance entre les corps plats (10a) du dispositif d'expansion par l'intermédiaire d'un élément de réglage à trou de serrure (12) formé d'une seule pièce,
- une paire de broches (13) insérées dans les corps plats (10a) du dispositif d'expansion de manière à ce que la vis d'extension (11) soit positionnée entre les broches (13) et conçue pour guider les déplacements des corps plats (10a) du dispositif d'expansion,
- quatre mini-vis (20), chaque mini-vis (20) avec une partie de tête et des filets externes sur ladite partie de tête, ce qui permet aux corps plats (10a) du dispositif d'expansion d'être fixés sur le palais de la mâchoire supérieure en étant fixés aux filets internes des trous de verrouillage (101), chaque trou de verrouillage de corps (101) étant situé entre une broche (13) et la vis d'extension (11) ;
- un tube de guidage (30) qui possède des filets externes sur sa surface externe afin d'être vissé dans les trous de verrouillage de corps (101) de manière à créer un chemin de guidage sur le palais en utilisant un foret à os approprié, puis dévissé, et qui possède une partie de tête (31) dont la surface supérieure s'étend à plat sur les côtés.

2. Dispositif selon la revendication 1, **caractérisé en ce que** la paire de corps plats (10a) du dispositif d'expansion est conçue pour être connectée à l'os situé à droite et à gauche avec une distance déterminée de la ligne médiane du palais par l'intermédiaire des trous de verrouillage de corps (101) et des mini-vis (20).
